(19) **Europäisches Patentamt / European Patent Office / Office européen des brevets**

(11) **EP 4 650 759 A1**

(12) # EUROPEAN PATENT APPLICATION

(43) Date of publication:
**19.11.2025  Bulletin 2025/47**

(21) Application number: **25174676.4**

(22) Date of filing: **07.05.2025**

(51) International Patent Classification (IPC):
***G01N 23/046*** (2018.01)

(52) Cooperative Patent Classification (CPC):
**G01N 23/046;** G01N 2223/419; G01N 2223/426;
G01N 2223/646; G01N 2223/6466

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority:  **10.05.2024  CN 202410580198**

(71) Applicant: **Siemens Healthineers AG
91301 Forchheim (DE)**

(72) Inventors:
• **CHEN, Wen Hao
Shanghai, 200010 (CN)**
• **TIAN, Yi
Shanghai, 200126 (CN)**
• **ZHANG, Li Dong
Shanghai, 201316 (CN)**
• **FAN, Liang Yan
Shanghai, 200331 (CN)**

(74) Representative: **HKW Intellectual Property PartG mbB
Theresienhöhe 12
80339 München (DE)**

(54) **METHOD FOR DETECTING ABNORMALITY IN MOVABLE COMPONENT IN OPTICAL PATH, COMPUTER PROGRAM, COMPUTER-READABLE STORAGE MEDIUM AND MEDICAL IMAGING DEVICE**

(57)  The present invention discloses a method for detecting an abnormality in a movable component in an optical path, a computer program, a computer-readable storage medium and medical imaging device. The method comprises: acquiring a difference $Diff(k, l, seg, r)$ between air correction charts with and without the movable component; determining a second difference $DIFF'(k, l, r)$ according to the difference $Diff(k, l, seg, r)$; subjecting the second difference $DIFF'(k, l, r)$ to low-pass filtering to obtain a smooth signal $S^{smooth}$; suppressing the influence of module response difference according to the smooth signal $S^{smooth}$ to obtain a reference signal $S(k, l, r)$; subjecting $S(k, l, r)$ to high-pass filtering in a channel direction to obtain a high-pass signal $S^{HP}$; comparing the high-pass signal $S^{HP}$ with a threshold $T$, to determine an abnormality in the movable component. The present invention utilizes existing air correction charts to comprehensively investigate abnormalities in movable components, without the need for additional scanning or image reconstruction.

S102

S103 → 100

S104

S106

S107

S108

S110

S112

S114

S116

Fig. 1

EP 4 650 759 A1

**Description**

In the present patent application, nouns and pronouns referring to people are not limited to a specific gender.

TECHNICAL FIELD

The present invention relates to the detection of abnormalities in movable components in optical paths, in particular an optical path in a medical imaging device.

BACKGROUND ART

In a computed tomography (CT) scan, the presence of abnormal components in the optical path will cause artifacts in the image. Examples of such abnormalities include processing defects, and deformations caused by knocks. Existing methods of inspecting optical path components are either directed at immovable components, or require additional scanning, perhaps even requiring the production of an image.

In addition, CT systems need to use an air correction chart to correct data obtained by scanning.

SUMMARY OF THE INVENTION

In view of the above, the present invention proposes a method for detecting an abnormality in a movable component in an optical path, a computer program, a computer-readable storage medium, and a medical imaging device.

According to a first aspect of the present invention, a method is provided for detecting an abnormality in a movable component in an optical path, the method comprising:

acquiring a difference *Diff (k, l, seg,* r) between air correction charts with and without the movable component, wherein *k* represents a channel, *l* represents a row, *sec* represents a partition, and *r* represents a focus position;

determining a second difference *DIFF'(k, l, r)* according to the difference *Diff(k, l, seg, r)*;

subjecting the second difference *DIFF'(k, l, r)* to low-pass filtering to obtain a smooth signal $S^{smooth}$;

suppressing the influence of module response difference according to the smooth signal $S^{smooth}$ to obtain a reference signal *S(k, l, r)*;

subjecting *S(k, l, r)* to high-pass filtering in a channel direction to obtain a high-pass signal $S^{HP}$;

comparing the high-pass signal $S^{HP}$ with a threshold *T,* to determine an abnormality in the movable component.

In an embodiment, the abovementioned steps comprise determining the second difference *DIFF'(k, l, r)* according to the following formula: *DIFF'(k, l, r) = Diff(k, l, seg, r)*.

In an embodiment, the abovementioned steps comprise: averaging the difference *Diff(k, l, seg, r)* in a partition dimension to obtain the second difference *DIFF'(k, l, r)*, *DIFF'(k, l, r) = mean(Diff,* 3), wherein *mean(Diff,* 3) represents averaging *Diff* in a third dimension.

In an embodiment, the abovementioned steps comprise: subjecting the difference *Diff(k, l, seg, r)* to numerical translation to obtain the second difference *DIFF'(k, l, r)*.

In an embodiment, the movable component is an ultra-high resolution comb.

In an embodiment, the abovementioned steps comprise subjecting the difference *Diff(k, l, seg, r)* to numerical translation according to the following formula:

$$DIFF'(k,l,r) = Diff(k,l,seg,r) + \sum_{i=k}^{N-1} shift1(k,r) \quad 1 \le k \le N-1$$

wherein:

$$shift1(k,r) = \begin{cases} shift0(k,r) & abs(shift0(k,r)) > T1 \\ 0 & else \end{cases}$$

$$shift0(k,r) = \frac{1}{N_l}\sum_l (Diff(k+1,l,seg,r) - Diff(k,l,seg,r)) \quad 1 \leq k \leq N-1$$

N is the number of channels in the same row of a detector,
$N_l$ is the number of rows, T1 is a threshold for determination, and *abs()* is an absolute value function.

In an embodiment, the abovementioned steps comprise:

performing edge extension on the second difference *DIFF'*, performing low-pass filtering, and cutting off an extension point to obtain a low-pass second difference *DIFF$^{Med}$*;
performing edge extension on the low-pass second difference *DIFF$^{Med}$*,
performing low-pass filtering, and cutting off an extension point to obtain a smooth signal *S$^{smooth}$*.

In an embodiment, the abovementioned steps comprise performing edge extension on the low-pass second difference *DIFF$^{Med}$*, according to the following formula and performing low-pass filtering:

$$DIFF_{Ext}^{Med} = Median(Ext(DIFF', EL0))$$

where *Median()* is a median function, and *Ext()* is an extension function, defined as follows:

$$Ext(P, EL) = \begin{cases} P(EL - k + 1, l, r) & k = 1{:}EL \\ P(k - EL, l, r) & k = EL + 1{:}N + EL \\ P(2*N + EL - k + 1, l, r) & k = N + EL + 1{:}N + 2*EL \end{cases}$$

where *N* is the number of channels in the same row of the detector, and *EL* is an extension length.

In an embodiment, the abovementioned steps comprise performing edge extension on the low-pass second difference *DIFF$^{Med}$*, according to the following formula and performing low-pass filtering:

$$S_{Ext1} = Ext(DIFF^{Med}, EL1)$$

$$S_{Ext1}^{smooth} = LP(S_{Ext1}, Nw1, Ns1)$$

where *LP (Q, Nw, Ns)* is a low-pass function, defined as follows:

$$LP(Q, Nw, Ns) = iFFT(ifftshift(fftshift(FFT(Q)).*W))$$

where FFT is a fast Fourier transform, iFFT is an inverse fast Fourier transform, fftshift is zero-frequency shift, ifftshift is inverse zero-frequency shift, and .* is a dot product operation;
a filter function *W* is defined as follows:

$$W(k)$$

$$= \begin{cases} 0 & k < \frac{N}{2} + EL - Nw + 1 \\ conv(k - (\frac{N}{2} + EL + 1))/\max(conv) & \frac{N}{2} + EL - Nw + 1 \leq k \leq \frac{N}{2} + EL + Nw + 1 \\ 0 & k > \frac{N}{2} + EL + Nw + 1 \end{cases}$$

$$conv(k) = \frac{e^{-0.5*(k*\frac{Ns}{Nw})^2}}{\sum_{i=-Nw}^{i=Nw} e^{-0.5*(i*\frac{Ns}{Nw})^2}}$$

*Ns* and *Nw* are parameters of a Gaussian function *conv(k)*;
*EL1* is a concrete instance of *EL*, *Ns*1 is a concrete instance of *Ns,* and *Nw*1 is a concrete instance of *Nw.*

In an embodiment, the abovementioned steps comprise obtaining the reference signal *S(k, l, r)* according to the following formula:

$$S(k,l,r) = \begin{cases} S^{smooth}(k,l,r) & 1 \leq k \leq CH \\ S^{smooth}(k,l,r) - shift2(ceil\left(\frac{k}{CH} - 1\right), l, r) & CH < k \leq N \end{cases}$$

$$shift2(m,l,r) = \sum_{n=1}^{m}(S^{smooth}(CH \cdot n + 1, l, r) - S^{smooth}(CH \cdot n, l, r)) \quad 1 \leq m < \frac{N}{CH}$$

where *CH* is the number of channels in the same row of the detector in a module, and *ceil* () is a ceiling function.

In an embodiment, the abovementioned steps comprise obtaining the high-pass signal $S^{HP}$ according to the following formula and steps:

$$S_{Ext2} = Ext(S, EL2)$$

$$S_{Ext2}^{LP} = LP(S_{Ext2}, Nw2, Ns2)$$

cutting off an extension point from $S_{Ext2}^{LP}$ to obtain a low-pass signal $S^{lp}$;

$$S^{HP} = S - S^{lp}$$

where *Ext()* is an extension function, defined as follows:

$$Ext(P, EL) = \begin{cases} P(EL - k + 1, l, r) & k = 1:EL \\ P(k - EL, l, r) & k = EL + 1:N + EL \\ P(2*N + EL - k + 1, l, r) & k = N + EL + 1:N + 2*EL \end{cases}$$

where *N* is the number of channels in the same row of the detector, and *EL* is an extension length;
*LP* (*Q, Nw, Ns*) is a low-pass function, defined as follows:

$$LP(Q, Nw, Ns) = iFFT(ifftshift(fftshift(FFT(Q)).*W))$$

where FFT is a fast Fourier transform, iFFT is an inverse fast Fourier transform, fftshift is zero-frequency shift, ifftshift is inverse zero-frequency shift, and .* is a dot product operation;
a filter function W is defined as follows:

$$W(k)$$

$$= \begin{cases} 0 & k < \dfrac{N}{2} + EL - Nw + 1 \\ conv(k - (\dfrac{N}{2} + EL + 1))/\max{(conv)} & \dfrac{N}{2} + EL - Nw + 1 \leq k \leq \dfrac{N}{2} + EL + Nw + 1 \\ 0 & k > \dfrac{N}{2} + EL + Nw + 1 \end{cases}$$

$$conv(k) = \frac{e^{-0.5*(k*\frac{Ns}{Nw})^2}}{\sum_{i=-Nw}^{i=Nw} e^{-0.5*(i*\frac{Ns}{Nw})^2}}$$

where $Ns$ and $Nw$ are parameters of a Gaussian function $conv(k)$;
$EL2$ is a concrete instance of $EL$, $Ns2$ is a concrete instance of $Ns$, and $Nw2$ is a concrete instance of $Nw$.

In an embodiment, the threshold $T$ is defined as follows:

$$T = \begin{cases} c & -f + d \leq k \leq f + d \\ a_1\sqrt{|k - d|} + a_2 & else \end{cases}$$

where $a_1 = \dfrac{b-c}{\sqrt{d-1}-\sqrt{f}}$, $a_2 = \dfrac{b\sqrt{f}-c\sqrt{d-1}}{-\sqrt{d-1}+\sqrt{f}}$, $b$ is a threshold of a detector edge, $c$ is a threshold of a detector center, $d$ is a channel ordinal number of the detector center, and $f$ is the number of channels in the same row at the left and right of the detector center with the threshold $c$.

According to a second aspect of the present invention, a computer program is provided which, when executed by a processor, can realize the steps of the method described above.

According to a third aspect of the present invention, a computer-readable storage medium with a computer program stored thereon is provided, wherein the program, when executed by a processor, can realize the steps of the method described above.

According to a fourth aspect of the present invention, a medical imaging device with an X-ray tube is provided, comprising the computer-readable storage medium described above.

The method for detecting an abnormality in a movable component in an optical path, the computer program, the computer-readable storage medium, and the medical imaging device of the present invention utilize existing air correction charts to comprehensively investigate abnormalities in movable components, without the need for additional scanning or image reconstruction, so can effectively prevent image quality issues due to component abnormalities. The present invention can check movable components one by one, to precisely indicate which component has an abnormality. This will facilitate troubleshooting, reducing final testing times on production lines, and reducing production line costs.

BRIEF DESCRIPTION OF THE DRAWINGS

Preferred embodiments of the present invention will be described in detail below with reference to the drawings, to give an ordinary person skilled in the art a clearer understanding of the abovementioned and other features and advantages of the present invention. In the drawings:

Fig. 1 is a schematic flow chart of a method for detecting an abnormality in a movable component in an optical path according to an embodiment of the present invention.

Fig. 2A is a schematic figure of an air correction chart with a movable component according to an embodiment of the present invention.

Fig. 2B is a schematic figure of an air correction chart without a movable component according to an embodiment of the present invention.

Fig. 2C is a schematic figure of the difference between the air correction chart of Fig. 2A and the air correction chart of Fig. 2B.

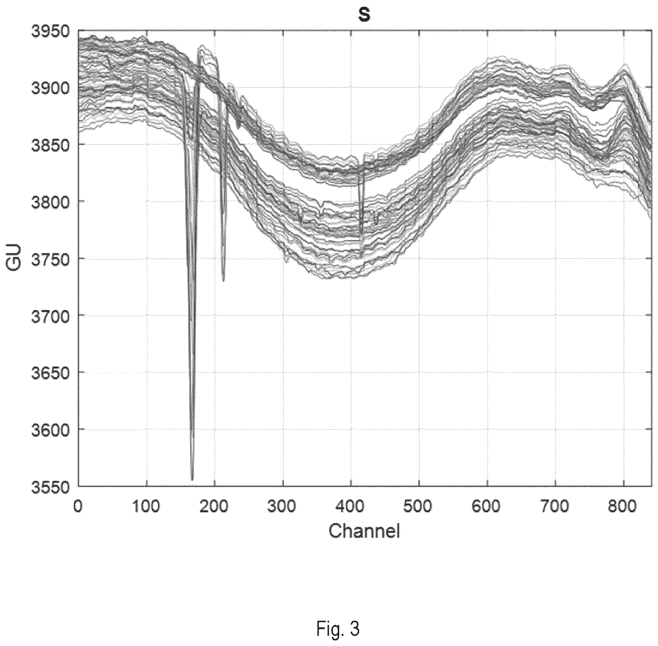

Fig. 3 is a schematic figure of a reference signal according to an embodiment of the present invention.

Fig. 4 is a schematic figure of a threshold according to an embodiment of the present invention.

Fig. 5 is a schematic figure
of a high-pass signal and a threshold according to an embodiment of the present invention.

## DETAILED DESCRIPTION OF EMBODIMENTS

To clarify the objectives, technical solutions and advantages of the present invention, the present invention will be explained in further detail below through embodiments.

An extension function $Ext(P, EL)$ for extension of $P$ in a channel direction is defined as follows:

$$Ext(P, EL) = \begin{cases} P(EL - k + 1, l, r) & k = 1:EL \\ P(k - EL, l, r) & k = EL + 1:N + EL \\ P(2 * N + EL - k + 1, l, r) & k = N + EL + 1:N + 2 * EL \end{cases}$$

wherein, in $P(k, l, r)$, $k$ represents a channel, $l$ represents a row, $r$ represents a focus position, $N$ is the number of channels in the same row of a detector, and $EL$ is an extension length.

A low-pass function $LP(Q, Nw, Ns)$ is defined as follows:

$$LP(Q, Nw, Ns) = iFFT(ifftshift(fftshift(FFT(Q)).* W))$$

where FFT is a fast Fourier transform, iFFT is an inverse fast Fourier transform, fftshift is shifting a zero-frequency component to the center (zero-frequency shift), ifftshift is inverse zero-frequency shift, and .* is a dot product operation. Matlab provides examples of fftshift and ifftshift, specifically:

Y = fftshift(X) rearranges a Fourier transform X by shifting the zero-frequency component to the center of an array.

If X is a vector, fftshift will exchange two halves (left and right) of X.

If X is a matrix, fftshift will exchange the first quadrant of X with the third quadrant, and exchange the second difference quadrant with the fourth quadrant.

If X is a multi-dimensional array, fftshift will exchange half-spaces of X along each dimension.

A filter function $W$ is defined as follows:

$$W(k)$$

$$= \begin{cases} 0 & k < \frac{N}{2} + EL - Nw + 1 \\ conv(k - (\frac{N}{2} + EL + 1))/\max(conv) & \frac{N}{2} + EL - Nw + 1 \leq k \leq \frac{N}{2} + EL + Nw + 1 \\ 0 & k > \frac{N}{2} + EL + Nw + 1 \end{cases}$$

$$conv(k) = \frac{e^{-0.5*(k*\frac{Ns}{Nw})^2}}{\sum_{i=-Nw}^{i=Nw} e^{-0.5*(i*\frac{Ns}{Nw})^2}}$$

$Ns$ and $Nw$ are parameters of a Gaussian function $conv(k)$; $Ns$ and $Nw$ influence the waveform distribution of the Gaussian function $conv(k)$.

In the present application, $EL1$ and $EL2$ are concrete instances of $EL$, $Ns1$ and $Ns2$ are concrete instances of $Ns$, and $Nw1$ and $Nw2$ are concrete instances of $Nw$.

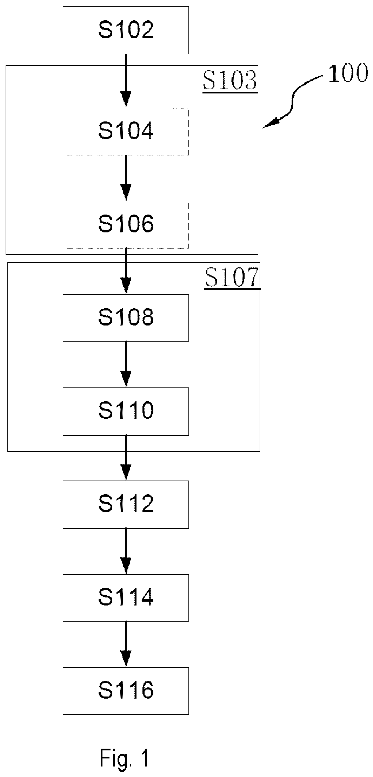

Fig. 1 is a schematic flow chart of a method 100 for detecting an abnormality in a movable component in an optical path according to an embodiment of the present invention. The movable component is for example a movable wedge filter, split filter or UHR comb. As shown in Fig. 1, the method 100 for detecting an abnormality in a movable component in an optical path comprises step S102, step S103, step S107, step S112, step S114 and step S116.

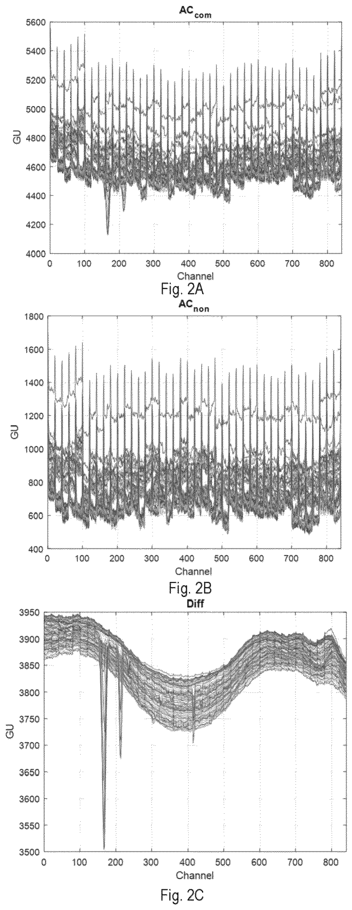

In step S102, the difference $Diff(k, l, seg, r)$ between air correction charts with and without the movable component is acquired. Fig. 2A is a schematic figure of an air correction chart $AC_{com}(k, l, seg, r)$ with a movable component according to an embodiment of the present invention. Fig. 2B is a schematic figure of an air correction chart $AC_{non}(k, l, seg, $ r) without a movable component according to an embodiment of the present invention. The air correction chart records a signal received by a detector in the course of an air scan. Here, $k$ represents a channel, $l$ represents a row, $sec$ represents a partition, and $r$ represents a focus position (focal spot index). For example, if there are two focus positions, then $r = 1$ may be used to represent focus position 1, and $r = 2$ may be used to represent focus position 2. A circumferential angle formed by rotation of the X-ray tube is evenly divided into partitions, and all angles within the same partition use an air correction chart of that partition. As shown in Figs. 2A and 2B, different curves represent different rows. Fig. 2C is a schematic figure of the difference $Diff(k, l, seg, r)$ between the air correction chart $AC_{com}(k, l, seg, r)$ of Fig. 2A and the air correction chart $AC_{non}(k, l, seg, $ r) of Fig. 2B, wherein $Diff(k, l, seg, r) = AC_{com}(k, l, seg, $ r) $- AC_{non}(k, l, seg, r)$. There is at least one air correction chart with a movable component and at least one air correction chart without a movable component in the X-ray optical path, and the difference $Diff(k, l, seg, r)$ may even have already been calculated.

In step S103, a second difference $DIFF'(k, l, r)$ is determined according to the difference $Diff(k, l, seg, r)$. The second difference $DIFF'(k, l, r)$ may be the same as the difference $Diff(k, l, seg, r)$. In an embodiment, preferably, before abnormality appraisal, to reduce noise, step S103 may further comprise step S104. In step S104, the difference $Diff(k, l, seg, r)$ is averaged in a partition dimension to obtain the second difference $DIFF'(k, l, r)$, $DIFF'(k, l, r) = mean(Diff, 3)$, wherein $mean(Diff, 3)$ represents averaging $Diff$ in a third dimension (partition). This averaging operation is not a requirement; in the present application, for conciseness, $DIFF'(k, l, r)$ may represent the average difference $mean(Diff, 3)$ or the difference $Diff(k, l, seg, r)$; these are collectively referred to as the second difference $DIFF'(k, l, r)$.

To reduce normal signal jumping caused by filters such as an ultra-high resonance comb (UHR comb), step S103 may further comprise step S106. In step S106, the difference $Diff(k, l, seg, r)$ is subjected to numerical translation (data shift) to obtain the second difference $DIFF'(k, l, r)$. In this embodiment, the expression for translation is;

$$DIFF'(k, l, r) = Diff(k, l, seg, r) + \sum_{i=k}^{N-1} shift1(k, r) \quad 1 \leq k \leq N - 1$$

wherein:

$$shift1(k, r) = \begin{cases} shift0(k, r) & abs(shift0(k, r)) > T1 \\ 0 & else \end{cases}$$

$$shift0(k, r) = \frac{1}{N_l} \sum_l (Diff(k + 1, l, seg, r) - Diff(k, l, seg, r)) \quad 1 \leq k \leq N - 1$$

N is the number of channels in the same row of a detector,
$N_l$ is the number of rows, T1 is a threshold for determination, and $abs()$ is an absolute value function. Numerical translation can avoid misjudgements caused by the abovementioned normal signal jumping.

It should be noted that the formula above actually calculates $DIFF'(k, l, sec, r)$, but for consistency of expression with other embodiments, the partition variable is omitted.

In other embodiments, depending on actual circumstances, numerical translation need not be performed. In other embodiments, step S103 may simultaneously comprise step S104 and step S106, performed one after the other. In this case, it is only necessary to replace $Diff(k, l, seg, r)$ on the right-hand side of the equation in step S106 with the result of step S104 (i.e. $DIFF'(k, l, r)$).

In step S107, the second difference $DIFF'(k, l, r)$ is subjected to low-pass filtering to obtain a smooth signal $S^{smooth}$. In this embodiment, step S107 may comprise step S108 and step S110.

Step S108: performing edge extension on the second difference $DIFF'$, performing low-pass filtering, and cutting off an extension point to obtain a low-pass second difference $DIFF^{Med}$. In this embodiment, a 5-fold median filter may be used, represented as follows:

$$DIFF_{Ext}^{Med} = Median(Ext(DIFF', EL0))$$

It can be seen from the formula above that edge extension is performed on *DIFF'* before median filtering, in order to keep the degrees of filtering of all points consistent.

An extension point is cut off from $DIFF_{Ext}^{Med}$ to obtain a low-pass second difference *DIFF^Med*.

Step S110: performing edge extension on the low-pass second difference *DIFF^Med*, performing low-pass filtering, and cutting off an extension point to obtain a smooth signal *S^smooth*. In this embodiment, the specific process is as follows:

Edge extension:

$$S_{Ext1} = Ext(DIFF^{Med}, EL1)$$

Low-pass filtering. In this embodiment, low-pass filtering is performed according to the following formula:

$$S_{Ext1}^{smooth} = LP(S_{Ext1}, Nw1, Ns1)$$

In other embodiments, a different low-pass filter may be used.

An extension point is cut off to obtain a smooth signal *S^smooth*.

Step S112: suppressing the influence of module response difference to obtain a reference signal S. In step S112, the following operation may be performed:

$$S(k,l,r) = \begin{cases} S^{smooth}(k,l,r) & 1 \leq k \leq CH \\ S^{smooth}(k,l,r) - shift2\left(ceil\left(\frac{k}{CH} - 1\right), l, r\right) & CH < k \leq N \end{cases}$$

$$shift2(m,l,r) = \sum_{n=1}^{m} (S^{smooth}(CH \cdot n + 1, l, r) - S^{smooth}(CH \cdot n, l, r)) \quad 1 \leq m < \frac{N}{CH}$$

where *CH* is the number of channels in the same row in a module of the detector, and *ceil* () is a ceiling function. *S* is a standard for detecting a component abnormality, so can be called a reference signal.

In *S(k, l, r)*, only the high-pass part indicates a component abnormality. Therefore, in step S114, *S(k, l, r)* is subjected to high-pass filtering in a channel direction to obtain a high-pass signal *S^HP*. In this embodiment, implementation is as follows:

$$S_{Ext2} = Ext(S, EL2)$$

$$S_{Ext2}^{LP} = LP(S_{Ext2}, Nw2, Ns2)$$

An extension point is cut off from $S_{Ext2}^{LP}$ to obtain a low-pass signal *S^lp*. Thus, the high-pass signal *S^HP* is calculated as follows:

$$S^{HP} = S - S^{lp}$$

In step S116, the high-pass signal *S^HP* is compared with a threshold T, to determine abnormalities in movable components. These abnormalities may include the presence or absence of an abnormality, whether an abnormality is acceptable, etc. The correlation of signal error to image quality varies with the square root of the distance to the center of the detector. Thus, in this embodiment, to reflect this phenomenon, a threshold *T* is defined as follows:

$$T = \begin{cases} c & -f + d \leq k \leq f + d \\ a_1\sqrt{|k - d|} + a_2 & else \end{cases}$$

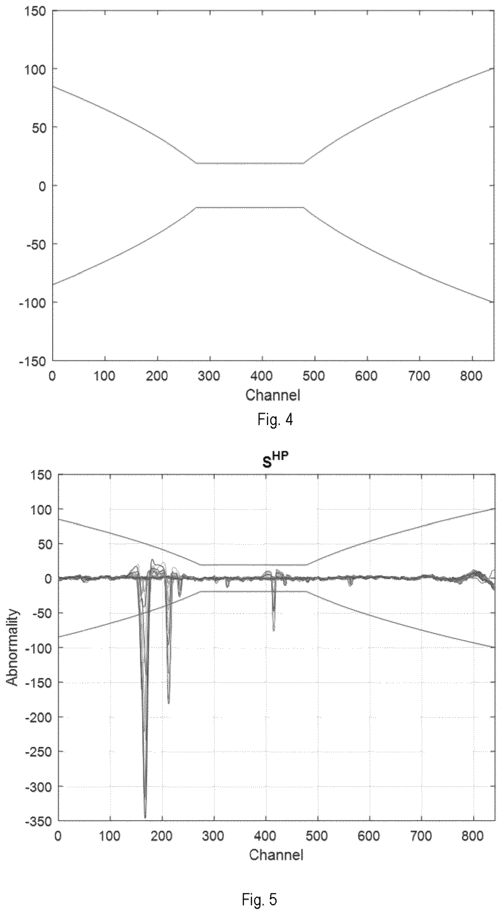

where $a_1 = \frac{b-c}{\sqrt{d-1}-\sqrt{f}}$, $a_2 = \frac{b\sqrt{f}-c\sqrt{d-1}}{-\sqrt{d-1}+\sqrt{f}}$, $b$ is a threshold of a detector edge, $c$ is a threshold of a detector center, $d$ is a channel ordinal number of the detector center, and $f$ is the number of channels in the same row at the left and right of the detector center with the threshold $c$. A channel with threshold c has total length 2f. If an abnormality represented by the high-pass signal $S^{HP}$ exceeds the threshold $T$, a warning is given that there is a high risk of image artifacts occurring due to a component abnormality. In other embodiments, the threshold $T$ may have other definitions. Fig. 4 is a schematic figure of a threshold $T$ according to an embodiment of the present invention. Fig. 5 is a schematic figure of a high-pass signal $S^{HP}$ and a threshold $T$ according to an embodiment of the present invention.

Before comparison with the threshold T: in the case of UHR mode, a constant weighting factor $w$1 will multiply the high-pass signal: in the case of a split filter, another weighting operation may be performed:

$$S_w^{HP} = S^{HP}(k, l, r) \cdot w(l)$$

The objective of the abovementioned weighting operation is to enable different movable components to use the same threshold $T$. Of course, if different thresholds are set for different movable components, then weighting need not be performed.

According to a second aspect of the present invention, a computer program is provided which, when executed by a processor, can realize the steps of the method 100 for detecting an abnormality in a movable component in an optical path.

According to a third aspect of the present invention, a computer-readable storage medium with a computer program stored thereon is provided, wherein the program, when executed by a processor, can realize the steps of the method 100 for detecting an abnormality in a movable component in an optical path.

According to a fourth aspect of the present invention, a medical imaging device with an X-ray tube is provided, comprising the computer-readable storage medium described above.

The method for detecting an abnormality in a movable component in an optical path, the computer program, the computer-readable storage medium, and the medical imaging device of the present invention utilize existing air correction charts to comprehensively investigate abnormalities in movable components, without the need for additional scanning or image reconstruction, so can effectively prevent image quality issues due to component abnormalities. The present invention can check movable components one by one, to precisely indicate which component has an abnormality. This will facilitate troubleshooting, reducing final testing times on production lines, and reducing production line costs.

The above are merely preferred embodiments of the present invention, which are not intended to limit it. Any amendments, equivalent substitutions or improvements etc. made within the spirit and principles of the present invention shall be included in the scope of protection thereof.

**Claims**

1. Method for detecting an abnormality in a movable component in an optical path, the method comprising:

   step S102, acquiring a difference $Diff(k, l, seg, r)$ between air correction charts with and without the movable component, wherein $k$ represents a channel, $l$ represents a row, $sec$ represents a partition, and $r$ represents a focus position;
   step S103, determining a second difference $DIFF'(k, l, r)$ according to the difference $Diff(k, l, seg, r)$;
   step S107, subjecting the second difference $DIFF'(k, l, r)$ to low-pass filtering to obtain a smooth signal $S^{smooth}$;
   step S112, suppressing the influence of module response difference according to the smooth signal $S^{smooth}$ to obtain a reference signal $S(k, l, r)$;
   step S114, subjecting $S(k, l, r)$ to high-pass filtering in a channel direction to obtain a high-pass signal $S^{HP}$;
   step S116, comparing the high-pass signal $S^{HP}$ with a threshold $T$, to determine an abnormality in the movable component.

2. Method according to Claim 1, **characterized in that** step S103 comprises determining the second difference $DIFF'(k, l, r)$ according to the following formula:

$$DIFF'(k, l, r) = Diff(k, l, seg, r).$$

3. Method according to Claim 1, **characterized in that** step S103 comprises:
   step S104, averaging the difference $Diff(k, l, seg, r)$ in a partition dimension to obtain the second difference $DIFF'(k, l, r)$, $DIFF'(k, l, r) = mean(Diff, 3)$, wherein $mean(Diff, 3)$ represents averaging $Diff$ in a third dimension.

4. Method according to Claim 1, **characterized in that** step S103 comprises:
   step S106: subjecting the difference $Diff(k, l, seg, r)$ to numerical translation to obtain the second difference $DIFF'(k, l, r)$.

5. Method according to Claim 4, **characterized in that** the movable component is an ultra-high resolution comb.

6. Method according to Claim 4, **characterized in that** step S106 comprises subjecting the difference $Diff(k, l, seg, r)$ to numerical translation according to the following formula:

$$DIFF'(k, l, r) = Diff(k, l, seg, r) + \sum_{i=k}^{N-1} shift1(k, r) \quad 1 \leq k \leq N - 1$$

wherein:

$$shift1(k, r) = \begin{cases} shift0(k, r) & abs(shift0(k, r)) > T1 \\ 0 & else \end{cases}$$

$$shift0(k, r) = \frac{1}{N_l} \sum_l (Diff(k + 1, l, seg, r) - Diff(k, l, seg, r)) \quad 1 \leq k \leq N - 1$$

N is the number of channels in the same row of a detector,
$N_l$ is the number of rows, T1 is a threshold for determination, and $abs()$ is an absolute value function.

7. Method according to Claim 1, **characterized in that** step S107 comprises step S108 and step S110;

   step S108: performing edge extension on the second difference $DIFF'$, performing low-pass filtering, and cutting off an extension point to obtain a low-pass second difference $DIFF^{Med}$;
   step S110: performing edge extension on the low-pass second difference $DIFF^{Med}$, performing low-pass filtering, and cutting off an extension point to obtain a smooth signal $S^{smooth}$.

8. Method according to Claim 7, **characterized in that** step S108 comprises performing edge extension on the low-pass second difference $DIFF^{Med}$, according to the following formula and performing low-pass filtering:

$$DIFF_{Ext}^{Med} = Median(Ext(DIFF', EL0))$$

where $Median()$ is a median function, and $Ext()$ is an extension function, defined as follows:

$$Ext(P, EL) = \begin{cases} P(EL - k + 1, l, r) & k = 1{:}EL \\ P(k - EL, l, r) & k = EL + 1{:}N + EL \\ P(2 * N + EL - k + 1, l, r) & k = N + EL + 1{:}N + 2 * EL \end{cases}$$

where $N$ is the number of channels in the same row of the detector, and $EL$ is an extension length.

9. Method according to Claim 8, **characterized in that** step S110 comprises performing edge extension on the low-pass second difference $DIFF^{Med}$, according to the following formula and performing low-pass filtering:

$$S_{Ext1} = Ext(DIFF^{Med}, EL1)$$

$$S_{Ext1}^{smooth} = LP(S_{Ext1}, Nw1, Ns1)$$

where *LP* (*Q, Nw, Ns*) is a low-pass function, defined as follows:

$$LP\ (Q, Nw, Ns) = iFFT(ifftshift(fftshift(FFT(Q)).* W))$$

where FFT is a fast Fourier transform, iFFT is an inverse fast Fourier transform, fftshift is zero-frequency shift, ifftshift is inverse zero-frequency shift, and .* is a dot product operation;
a filter function *W* is defined as follows:

$$W(k)$$

$$= \begin{cases} 0 & k < \frac{N}{2} + EL - Nw + 1 \\ conv(k - (\frac{N}{2} + EL + 1))/\max{(conv)} & \frac{N}{2} + EL - Nw + 1 \leq k \leq \frac{N}{2} + EL + Nw + 1 \\ 0 & k > \frac{N}{2} + EL + Nw + 1 \end{cases}$$

$$conv(k) = \frac{e^{-0.5*(k*\frac{Ns}{Nw})^2}}{\sum_{i=-Nw}^{i=Nw} e^{-0.5*(i*\frac{Ns}{Nw})^2}}$$

*Ns* and *Nw* are parameters of a Gaussian function *conv(k)*;
*EL1* is a concrete instance of *EL, Ns1* is a concrete instance of *Ns,* and *Nw*1 is a concrete instance of *Nw.*

**10.** Method according to Claim 1, **characterized in that** step S112 comprises obtaining the reference signal *S(k, l, r)* according to the following formula:

$$S(k, l, r) = \begin{cases} S^{smooth}(k, l, r) & 1 \leq k \leq CH \\ S^{smooth}(k, l, r) - shift2(ceil\left(\frac{k}{CH} - 1\right), l, r) & CH < k \leq N \end{cases}$$

$$shift2(m, l, r) = \sum_{n=1}^{m} (S^{smooth}(CH \cdot n + 1, l, r) - S^{smooth}(CH \cdot n, l, r)) \quad 1 \leq m < \frac{N}{CH}$$

where *CH* is the number of channels in the same row in a module of the detector, and *ceil* () is a ceiling function.

**11.** Method according to Claim 1, **characterized in that** step S114 comprises obtaining the high-pass signal $S^{HP}$ according to the following formula and steps:

$$S_{Ext2} = Ext(S, EL2)$$

$$S_{Ext2}^{LP} = LP(S_{Ext2}, Nw2, Ns2)$$

cutting off an extension point from $S^{LP}_{Ext2}$ to obtain a low-pass signal $S^{lp}$;

$$S^{HP} = S - S^{lp}$$

where *Ext()* is an extension function, defined as follows:

$$Ext(P, EL) = \begin{cases} P(EL - k + 1, l, r) & k = 1:EL \\ P(k - EL, l, r) & k = EL + 1:N + EL \\ P(2 * N + EL - k + 1, l, r) & k = N + EL + 1:N + 2 * EL \end{cases}$$

where *N* is the number of channels in the same row of the detector, and *EL* is an extension length;
*LP* (*Nw, Ns*) is a low-pass function, defined as follows:

$$LP\,(Q, Nw, Ns) = iFFT(ifftshift(fftshift(FFT(Q)).* W))$$

where FFT is a fast Fourier transform, iFFT is an inverse fast Fourier transform, fftshift is zero-frequency shift, ifftshift is inverse zero-frequency shift, and .* is a dot product operation;
a filter function *W* is defined as follows:

$$W(k)$$

$$= \begin{cases} 0 & k < \frac{N}{2} + EL - Nw + 1 \\ conv(k - (\frac{N}{2} + EL + 1))/\max(conv) & \frac{N}{2} + EL - Nw + 1 \le k \le \frac{N}{2} + EL + Nw + 1 \\ 0 & k > \frac{N}{2} + EL + Nw + 1 \end{cases}$$

$$conv(k) = \frac{e^{-0.5*(k*\frac{Ns}{Nw})^2}}{\sum_{i=-Nw}^{i=Nw} e^{-0.5*(i*\frac{Ns}{Nw})^2}}$$

where *Ns* and *Nw* are parameters of a Gaussian function *conv(k)*;
*EL2* is a concrete instance of *EL, Ns2* is a concrete instance of *Ns,* and *Nw2* is a concrete instance of *Nw.*

**12.** Method according to Claim 1, **characterized in that** the threshold T is defined as follows:

$$T = \begin{cases} c & -f + d \le k \le f + d \\ a_1\sqrt{|k - d|} + a_2 & else \end{cases}$$

where $a_1 = \frac{b-c}{\sqrt{d-1}-\sqrt{f}}$, $a_2 = \frac{b\sqrt{f}-c\sqrt{d-1}}{-\sqrt{d-1}+\sqrt{f}}$, *b* is a threshold of a detector edge, *c* is a threshold of a detector center, *d* is a channel ordinal number of the detector center, and *f* is the number of channels in the same row at the left and right of the detector center with the threshold *c.*

**13.** Computer program which, when executed by a processor, can realize the steps of the method according to any one of Claims 1 - 12.

**14.** Computer-readable storage medium with a computer program stored thereon, **characterized in that** the program, when executed by a processor, can realize the steps of the method according to any one of Claims 1 - 12.

15. Medical imaging device with an X-ray tube, the medical imaging device comprising the computer-readable storage medium according to Claim 14.

Fig. 1

Fig. 2A

Fig. 2B

Fig. 2C

Fig. 3

Fig. 4

Fig. 5

**EUROPEAN SEARCH REPORT**

Europäisches
Patentamt
European
Patent Office
Office européen
des brevets

**Application Number**

EP 25 17 4676

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Zhang Guoqing ET AL: "Biomedical Physics & Engineering Express Correction of Bowtie filter induced scatter signals based on air scan data and object scan data", Biomed. Phys. Eng. Express, 28 June 2022 (2022-06-28), pages 1-10, XP055983143, Retrieved from the Internet: URL:https://iopscience.iop.org/article/10.1088/2057-1976/ac5d0c/pdf [retrieved on 2022-11-19] * Abstract; section 2.1 entitled "Determination of scatter signals"; pages 3, 5; figure 1 * | 1-15 | INV. G01N23/046 |
| Y | US 2016/253818 A1 (TANG QIULIN [US] ET AL) 1 September 2016 (2016-09-01) * the whole document * | 1-15 | |
| A | DIKAIOS NIKOLAOS ET AL: "Improved motion-compensated image reconstruction for PET using sensitivity correction per respiratory gate and an approximate tube-of-response backprojector", MEDICAL PHYSICS, AIP, MELVILLE, NY, US, vol. 38, no. 9, 1 September 2011 (2011-09-01), pages 4958-4970, XP012151144, ISSN: 0094-2405, DOI: 10.1118/1.3611041 [retrieved on 2011-08-09] * the whole document * | 1-15 | **TECHNICAL FIELDS SEARCHED (IPC)** G01N |
| A | US 2015/103972 A1 (BREDNO JOERG [US] ET AL) 16 April 2015 (2015-04-16) * the whole document * | 1-15 | |

-/--

The present search report has been drawn up for all claims

| Place of search | Date of completion of the search | Examiner |
|---|---|---|
| The Hague | 9 October 2025 | Martin, Hazel |

page 1 of 2

Europäisches
Patentamt

European
Patent Office

Office européen
des brevets

## EUROPEAN SEARCH REPORT

**Application Number**

EP 25 17 4676

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JP 2015 000231 A (TOSHIBA CORP; TOSHIBA MEDICAL SYS CORP) 5 January 2015 (2015-01-05) * the whole document * | 1-15 | |

-----

TECHNICAL FIELDS
SEARCHED      (IPC)

The present search report has been drawn up for all claims

| Place of search | Date of completion of the search | Examiner |
|---|---|---|
| The Hague | 9 October 2025 | Martin, Hazel |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

..............................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 17 4676

This annex lists the patent family members relating to the patent documents cited in the above-mentioned European search report.
The members are as contained in the European Patent Office EDP file on
The European Patent Office is in no way liable for these particulars which are merely given for the purpose of information.

09-10-2025

| Patent document cited in search report | Publication date | Patent family member(s) | | Publication date |
|---|---|---|---|---|
| US 2016253818 A1 | 01-09-2016 | JP | 6691793 B2 | 13-05-2020 |
| | | JP | 2016159156 A | 05-09-2016 |
| | | US | 2016253818 A1 | 01-09-2016 |
| US 2015103972 A1 | 16-04-2015 | CN | 104334081 A | 04-02-2015 |
| | | EP | 2854645 A1 | 08-04-2015 |
| | | JP | 6316283 B2 | 25-04-2018 |
| | | JP | 2015518765 A | 06-07-2015 |
| | | RU | 2014154006 A | 10-08-2016 |
| | | US | 2015103972 A1 | 16-04-2015 |
| | | WO | 2013182928 A1 | 12-12-2013 |
| JP 2015000231 A | 05-01-2015 | JP | 6334853 B2 | 30-05-2018 |
| | | JP | 2015000231 A | 05-01-2015 |

EPO FORM P0459

For more details about this annex : see Official Journal of the European Patent Office, No. 12/82